(19) Europäisches Patentamt
European Patent Office
Office européen des brevets

(11) EP 1 600 532 B1

(12) **EUROPÄISCHE PATENTSCHRIFT**

(45) Veröffentlichungstag und Bekanntmachung des Hinweises auf die Patenterteilung:
**13.06.2007 Patentblatt 2007/24**

(51) Int Cl.:
***D01F 1/10*** *(2006.01)* ***C11C 3/00*** *(2006.01)*
***D06M 13/17*** *(2006.01)* ***D06M 15/53*** *(2006.01)*

(21) Anmeldenummer: **04012467.9**

(22) Anmeldetag: **26.05.2004**

(54) **Hydrophilierung von Polyolefin und/oder Polyester enthaltenden Materialien**

Hydrophilising of polyolefin- and/or polyester-containing materials

Hydrophilisation de materiaux comportant des polyolefins et/ou des polyesters

(84) Benannte Vertragsstaaten:
**AT BE BG CH CY CZ DE DK EE ES FI FR GB GR HU IE IT LI LU MC NL PL PT RO SE SI SK TR**

(43) Veröffentlichungstag der Anmeldung:
**30.11.2005 Patentblatt 2005/48**

(73) Patentinhaber: **Dr. Th. Böhme KG Chem. Fabrik GmbH & Co.**
**82538 Geretsried (DE)**

(72) Erfinder:
- **Warncke, Wolfgang**
**83646 Wackersberg (DE)**
- **Häner, Emma**
**82538 Geretsried (DE)**
- **Karabed, Martina**
**82515 Wolfratshausen (DE)**

(74) Vertreter: **Geyer, Fehners & Partner Patentanwälte**
**Perhamerstraße 31**
**80687 München (DE)**

(56) Entgegenhaltungen:
**EP-A- 0 254 547** **EP-A- 0 547 846**
**EP-A- 0 586 323** **DE-A1- 1 934 540**
**DE-A1- 19 851 688** **US-A- 5 362 894**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 600 532 B1

Printed by Jouve, 75001 PARIS (FR)

## Beschreibung

Die Erfindung bezieht sich auf die Verwendung einer speziellen Verbindung zur permanenten Hydrophilierung von Polyolefin und/oder Polyester enthaltenden Materialien.

In zahlreichen Fällen muß die Oberfläche von Kunststofferzeugnissen mit speziellen Effekten versehen werden, die sich während der Formgebung entweder aus technischen Gründen gar nicht bzw. nur unvollkommen oder aber aus wirtschaftlichen Gründen nur unvorteilhaft erzeugen lassen. Ein solcher Effekt ist beispielsweise die Verbesserung der Benetzbarkeit mit polaren Flüssigkeiten, wie Wasser. Technische Anwendungen dafür liegen beispielsweise auf dem Gebiet der Herstellung von Hygieneartikeln.

Bei der Herstellung von Hygieneartikeln, wie Windeln oder Damenbinden, werden absorbierende Materialien verwendet, um wäßrige Flüssigkeiten aufzunehmen. Um den direkten Kontakt mit dem absorbierenden Material beim Tragen zu verhindern und den Tragekomfort zu erhöhen, wird dieses Material mit einem dünnen, wasserdurchlässigen Vliesstoff umhüllt. Derartige Vliesstoffe werden üblicherweise aus synthetischen Fasern, wie Polyolefin- oder Polyesterfasern hergestellt, da diese Fasern preiswert zu produzieren sind, gute mechanische Eigenschaften aufweisen und thermisch belastbar sind. Allerdings eigenen sich unbehandelte Polyolefin- oder Polyesterfasern für diesen Einsatzzweck nicht, da sie aufgrund ihrer hydrophoben Oberfläche keine ausreichende Durchlässigkeit für wäßrige Flüssigkeiten aufweisen.

Bisher wurde die Hydrophilierung durch Einsatz von stark oberflächenaktiven Substanzen erreicht. Bedingt durch neue Windelkonstruktionen haben sich aber auch die Anforderungen an die Vliese und deren Avivagen verändert. Die sogenannten "permanenten" Präparationen lassen mehrere Befeuchtungen zu, ohne naß zu wirken. Dazu darf die Präparation nur noch sehr schwach oberflächlich aktiv sein.

Es ist prinzipiell möglich, die Fasern durch nachträgliches Beschichten mit entsprechenden Präparationen die nötigen hydrophilen Eigenschaften zu verleihen oder bereits durch Zusatz geeigneter innerer Additive bei der Herstellung der Fasern diese ausreichend hydrophil auszurüsten.

Die DE 100 15 554 A1 beschreibt Additive zur permanenten Hydrophilierung von Polyolefin enthaltenden Materialien, vorzugsweise Polyproylenfasern, wobei das Umsetzungsprodukt von ein Teil Polyethylenglykol mit zwei Teilen Fettsäuren mit 10 bis 12 C-Atomen eingesetzt wird.

Die aus dem Stand der Technik bekannten Hydrophilierungsmittel sind allerdings hinsichtlich Permanenz und Hydrophilie noch verbesserungswürdig. Des weiteren ist an ihnen nachteilig, daß sie erst in aufwendiger Weise in Lösung gebracht werden müssen, bevor sie eingesetzt werden können. Für die Applikation sind die aus dem Stand der Technik bekannten Hydrophilierungsmittel also ungünstig.

Der vorliegenden Erfindung liegt somit die Aufgabe zugrunde, die Hydrophilierung von Polyolefin und/oder Polyester enthaltenden Materialien ohne die aus dem Stand der Technik bekannten Nachteile zu ermöglichen.

Erfindungsgemäß wird dies erreicht durch die Verwendung einer Verbindung der Formel (1).

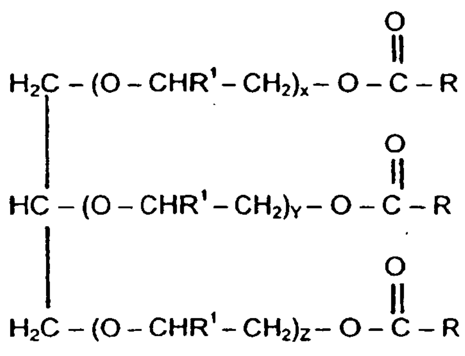

(1)

in der
die Reste $R^1$ gleich oder verschieden und unabhängig voneinander ausgewählt sind unter H und $CH_3$;
die Reste R gleich oder verschieden und unabhängig voneinander ausgewählt sind unter C7-C19-Fettsäureresten, und

$$x + y + z = 5 - 50,$$

zur permanenten Hydrophilierung von Polyolefin und/oder Polyester enthaltenden Materialien.

Die Summe der in der erfindungsgemäß verwendeten Verbindung vorliegenden Bestandteile (O-$CHR^1$-$CH_2$)

beträgt 5 bis 50, insbesondere 10 bis 30. Es ist dabei günstig, wenn jede der drei OH-Gruppen des Glycerin-Rests mindestens einen Rest (O-$CHR^1$-$CH_2$) aufweist.

Die Reste (O-$CHR^1$-$CH_2$) können von Ethylenoxid oder Propylenoxid oder Gemischen davon abgeleitet sein. Die Zusammensetzung der Reste (O-$CHR^1$-$CH_2$) in der erfindungsgemäß verwendeten Verbindung hängt dabei von Art und Zusammensetzung der Alkylenoxide ab, die zur Herstellung eingesetzt werden. Wird beispielsweise Glycerin mit Ethylenoxid umgesetzt, so weist die erfindungsgemäß verwendete Verbindung (O-$CH_2$-$CH_2$)-Einheiten auf. Läßt man Glycerin mit Propylenoxid reagieren, werden (O-$CHCH_3$-$CH_2$)-Einheiten erhalten. Wird ein Gemisch von Ethylenoxid und Propylenoxid eingesetzt, so liegen von Ethylenoxid und von Propylenoxid abgeleitete Einheiten in der erfindungsgemäßen Verbindung statistisch verteilt vor.

In der erfindungsgemäß verwendeten Verbindung liegen drei Reste R vor, die von C8-C20-Fettsäuren der Formel R-$CO_2H$ abgleitet sind. Bei Fettsäuren handelt es sich um eine Gruppenbezeichnung für aliphatische, gesättigte oder ungesättigte, insbesondere einfach ungesättigte, Fettsäuren, die verzweigt oder unverzweigt sein können. Fettsäuren haben die allgemeine Formel R-COOH. Dabei stammt R der erfindungsgemäß verwendeten Verbindung von der allgemeinen Formel R-COOH der Fettsäuren. R ist dabei insbesondere ein gesättigter unverzweigter oder einfach ungesättigter, unverzweigter Rest. Die Anzahl der C-Atome des Restes R beträgt insbesondere 11 bis 17 in der Verbindung.

Die drei Reste R der erfindungsgemäß verwendeten Verbindung können gleich oder verschieden voneinander sein. Wird bei der Herstellung der erfindungsgemäß verwendeten Verbindung nur eine Art von Fettsäure eingesetzt, sind alle drei Reste R in der erfindungsgemäß verwendeten Verbindung identisch. Wird zur Herstellung der erfindungsgemäß verwendeten Verbindung jedoch ein Gemisch von Fettsäuren verwendet, sind die Reste R der erfindungsgemäß verwendeten Verbindung abhängig von der Zusammensetzung des Fettsäuregemisches unterschiedlich.

In einer bevorzugten Ausführungsform der erfindungsgemäß verwendeten Verbindung stammt der Fettsäurerest R von Ölsäure, Laurinsäure, iso-Stearinsäure oder Kokosfettsäure, wobei letzteres die Bezeichnung für ein Gemisch ist, das im wesentlichen Laurin- und Myristinsäure enthält. Diese Reste R sind im Hinblick auf Permanenz und Hydrophilie vorteilhaft.

Die erfindungsgemäß verwendeten Verbindung zeichnet sich durch eine Reihe von Vorteilen aus. Sie können in einfacher, schneller und kostengünstiger Weise hergestellt werden. Sie haben hinsichtlich Permanenz und Hydrophilie hervorragende Eigenschaften. Sie sind anwendungstechnisch besonders einfach zu handhaben. Sie können ohne großen mechanischen Aufwand und ohne Anwendung erhöhter Temperaturen vor der Anwendung gelöst werden, so daß die Verwendung der Verbindungen in automatischen Verdünnungs- und Dosieranlagen ohne Probleme möglich ist.

Zur Herstellung der erfindungsgemäß verwendeten Verbindung wird eine Verbindung der Formel (2)

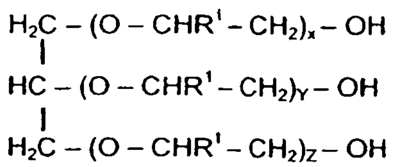

(2),

mit einer C8-C20-Fettsäure R-$CO_2H$ verestert wird, wobei $R^1$, R und x, y und z die vorstehend angegebenen Bedeutungen haben.

Zur Herstellung der erfindungsgemäß verwendeten Verbindungen wird von dem Umsetzungsprodukt aus Glycerin und Ethylenoxid oder Propylenoxid oder einem Gemisch davon ausgegangen. Dieses Umsetzungsprodukt weist freie Hydroxyl-Gruppen auf, die mit den Fettsäuren verestert werden können.

Die Veresterung kann unter Einsatz eines sauren Katalysators, beispielsweise p-Toluolsulfonsäure, erfolgen. Es ist günstig, die Veresterungsreaktion ohne Lösungsmittel durchzuführen, da dann die Abtrennung der Verbindung nicht notwendig ist.

Die Veresterungsreaktion wird so lange durchgeführt, bis eine vollständige Umsetzung aller OH-Gruppen mit der Säure erfolgt ist. Dies ist beispielsweise dann der Fall, wenn kein Wasser mehr abgeschieden wird, was in üblicher Weise bestimmt werden kann. Bis zur Vervollständigung der Veresterung beträgt die Reaktionsdauer 4 bis 6 Stunden. Die Reaktionstemperatur beträgt etwa 190°C bis 200°C.

Nach der Herstellung der erfindungsgemäß verwendeten Verbindung in der vorstehend beschriebenen Weise ist eine weitere Reinigung der Verbindung nicht notwendig. Als günstig hat es sich herausgestellt, daß Reaktionsgemisch noch zu neutralisieren, beispielsweise durch Zugabe einer starken Lauge, wie Natrium- oder Kalilauge, oder eines organischen Amins. Vor dem Einsatz als Hydrophilierungsmittel kann noch Wasser und/oder Stellmittel, beispielsweise Glykol, letzteres in einer Menge von nicht mehr als etwa 5 Gew.-%, zugegeben werden. Durch dieses Verfahren können die erfindungsgemäß verwendeten Verbindungen in einfacher, schneller und kostengünstiger Weise hergestellt werden.

Die erfindungsgemäß verwendeten Verbindungen sind aufgrund ihrer Eigenschaften bestens zur Verwendung zur permanenten Hydrophilierung von Polyolefin und/oder Polyester enthaltenden Materialien geeignet.

Das Polyolefin ist dabei vorzugsweise ein Homo- oder Copolymer auf Ethylen- oder Propylen-Basis. Beispiele besonders geeigneter Poiymertypen sind in der nachfolgenden Zusammenstellung aufgezählt:

Poly(ethylene) wie HDPE (high density polyethylene), LDPE (low density polyethylene), VLDPE (very low density polyethylene), LLDPE (linear low density polyethylene), MDPE (medium density polyethylene), UHMPE (ultra high molecular polyethylene), VPE (vernetztes Polyethylen), HPPE (high pressure polyethylene); Poly(propylene) wie isotaktisches Polypropylen; syndiotaktisches Polypropylen, Metallocen-katalysiert hergestelltes Propylen, schlagzähmodifiziertes Polypropylen, Random-Copolymere auf Basis von Ethylen und Propylen, Blockcopolymere auf Basis von Ethylen und Propylen; EPM (Poly[ethylen-co-propylen]); EPDM (Poly[ethylen-co-propylen-cokonjugiertes Dien]). Weitere geeignete Polymertypen sind: Poly(styrol); Poly(methylstyrol); Poly(oxymethylen); Metallocenkatalysierte alpha-Olefin- oder Cycloolefin-Copolymere wie Norbornen-Ethylen-Copolymere; Copolymere, die zu mindestens 60% Ethylen und/oder Styrol enthalten und zu weniger als 40% Monomere wie Vinylacetat, Acrylsäureester, Methacrylsäureester, Acrylsäure, Acrylnitril, Vinylchlorid. Beispiele solcher Polymeren sind: Poly(ethylen-co-ethylacrylat), Poly(ethylen-co-vinylacetat), Poly(ethylen-co-vinylchlorid), Poly(styrol-co-acrylnitril). Geeignet sind weiterhin Pfropfcopolymere sowie Polymerblends, daß heißt, Mischungen von Polymeren, in denen unter anderem die vorgenannten Polymere enthalten sind, beispielsweise Polymerblends auf Basis von Polyethylen und Polypropylen.

Im Rahmen der vorliegenden Erfindung sind Homo- und Copolymere auf Basis von Ethylen und Propylen besonders bevorzugt. In einer Ausführungsform der vorliegenden Erfindung setzt man dementsprechend als Polyolefin ausschließlich Polyethylen ein, in einer anderen Ausführungsform ausschließlich Polypropylen, in einer weiteren Ausführungsform Copolymere auf Basis von Ethylen und Propylen.

Vertreter des Polyesters ist der Ester aus Terephthalsäure und Diethylenglykol.

In einer anderen bevorzugten Ausführungsform der erfindungsgemäßen Verwendung ist das Material eine Faser oder ein Flächengebilde. Günstigerweise sind dabei die textilen Flächengebilde Vliesstoffe, insbesondere solche, die zum Einsatz in Windeln bestimmt sind.

Bei der erfindungsgemäßen Anwendung kann die erfindungsgemäß verwendete Verbindung sowohl auf die fertige Faser als auch bei der Faserherstellung zur Polymermasse zugegeben werden. Für beide Verfahren kennt der Fachmann die notwendigen Schritte, Materialien und Vorrichtungen.

Wird die erfindungsgemäß verwendete Verbindung auf die fertige Faser appliziert, so kann die Menge der Verbindung 0,1 g bis 2,0 g, insbesondere etwa 0,5 g, bezogen auf 100 g Faser, betragen. Wird die erfindungsgemäß verwendete Verbindung bei der Faserherstellung zugesetzt, so kann die Menge etwa 5 Gew.-%, bezogen auf die Polymermenge, betragen.

Die erfindungsgemäß verwendete Verbindung ist insbesondere für solche Faserpolymere geeignet, die beim Kontakt mit Wasser nur ein geringes Auswaschen der Wirkkomponente, d.h. der Verbindung, zeigen dürfen. Dies bedeutet also, daß die erfindungsgemäß verwendete Verbindung sich nur sehr schwer und somit in sehr geringen Mengen aus den Fasermaterialien auswaschen läßt.

Ein weiteres Einsatzgebiet bei der erfindungsgemäßen Verwendung sind Polyester und Polypropylenspunbond und Stapelfasern im Hygieneeinsatz.

Die nachfolgenden Beispielen sollen die Erfindung näher erläutern, ohne sie jedoch einzuschränken.

**Herstellungsbeispiele:**

1. Muster **M1:** 67,3g Glycerin x 26 mol Ethlyenoxid (EO) werden mit 32,7g Laurinsäure umgesetzt. Als Katalysator werden 0,3g para-Toluolsulfonsäure zugegeben. Nach 6 Stunden Umsetzung bei 200°C wird kein Wasser mehr abgeschieden. Die Umsetzung ist beendet, was durch Bestimmung der Säurezahl (SZ) des Gemisches überprüft werden kann (SZ < 12 mg KOH/g).

2. Muster **M2:** 60,6g Glycerin x 26 mol EO werden mit 39,4g Ölsäure umgesetzt. Als Katalysator werden 0,3g para-Toluolsulfonsäure zugegeben. Nach 5 Stunden Umsetzung bei 190°C wird kein Wasser mehr abgeschieden. Die Säurezahl des Gemisches ist kleiner als 10 mg KOH/g.

3. Muster **M3**: 51,1g Glycerin x 12 mol EO werden mit 48,9g Kokosfettsäure umgesetzt. Als Katalysator werden 0,3g para-Toluolsulfonsäure zugegeben. Nach 5 Stunden Umsetzung bei 200°C wird kein Wasser mehr abgeschieden. Die Säurezahl des Gemisches ist kleiner als 10 mg KOH/g.

**Anwendungsbeispiele:**

Zur Bestimmung der hydrophilen Eigenschaften werden die erfindungsgemäßen Verbindungen **M1, M2** und **M3** auf Polypropylen Spunbond-Vlies mit dem Flächengewischt 17 g/m$^2$ aufgetragen. Die Vliese werden nach Konditionierung bei 20°C und 65% relativer Luftfeuchte getestet.

Auflagehöhe

Die Auflagehöhe gibt an, wie viel Präparation sich auf einem Polymermaterial befindet. Sie wird durch Extraktion mit einem geeigneten Lösemittel bestimmt und in Masse % angegeben.

Mindestlösetemperatur

Das Aufbringen der Präparationen auf die Oberfläche der Vliese oder Fasern erfolgt über eine wäßrige Lösung. Die Mindestlösetemperatur gibt an, welche Temperatur das zur Verdünnung verwendete Wasser mindestens haben muß, um eine homogene Mischung zu erhalten. Beim Test wird Wasser der angegebenen Temperatur langsam unter Rühren zu dem mit -25°C vorgelegten Produkt gegeben. Je niedriger die Mindestlösetemperatur, desto einfacher ist der Umgang mit der Präparation.

Improved strike through EDANA 153.0-02

Synthetischer Urin (5 ml) werden im Testgerät nacheinander appliziert. Die Flüssigkeit muß vollständig durch das Vlies laufen. Es wird jeweils die Zeit gestoppt, die für den Flüssigkeitsdurchtritt benötigt wird.

Repeated Run-off EDANA 152.0-99

Synthetischer Urin (25 ml) werden mehrfach über einen Sandwich aus Vliesprobe und Filterpapier ausgegossen. Die Probe ist 25° gegen die Horizontale geneigt. Gemessen wird die Menge an synthetischem Urin, die über eine Strecke von 250 mm hinausläuft. Die Angabe erfolgt in % bezogen auf die eingesetzten 25 ml. Dieser Test reagiert empfindlicher auf die Hydrophilie der Präparation, da gegenüber dem Test "Improved strike through" keine mechanische Flüssigkeitssäule aufgebaut wird.

Vergleichsmuster

Muster MV4 ist eine handelsübliche Präparation (quartäre Ammoniumverbindung auf Basis von Steorylamin) zur Herstellung permanent hydrophiler Vliese.

| Prüfmaterial PP Spunbond Vlies 17 g/m$^2$ | | | | | | | | |
|---|---|---|---|---|---|---|---|---|
| Muster | Auflagehöhe | Mindestlöse-temperatur | Improved strike through | | | Repeated Run-off | | |
| # | % | °C | 1. s | 2. s | 3. s | 1. % | 2. % | 3. % |
| M1 | 0,5 | 30 | 1,8 | 2,7 | 3,7 | 6 | 0 | 13 |
| M2 | 0,5 | 30 | 2,0 | 2,7 | 2,9 | 34 | 5 | 9 |
| M3 | 0,5 | 30 | 1,4 | 2,6 | 3,3 | 3 | n.b. | n.b. |
| MV4 | 0,5 | 60 | 1,4 | 2,4 | 4,1 | 2 | n.b. | n.b. |
| n.b. nicht bestimmt | | | | | | | | |

Die Substanzen stellen Wirkstoffe für permanent hydrophile Präparationen dar, die für leicht aufzulösende Produkte verwendet werden können. Damit wird ein großer Nachteil der bisher bekannten Produkte aufgehoben. Bisher mußten die Präparationen für permanent hydrophile Ausrüstung unter hohem mechanischem Aufwand und unter Anwendung erhöhter Temperaturen vor der Anwendung gelöst werden. Dieser Aufwand reduziert sich jetzt. Damit wird die Benutzung bestehender automatischer Verdünnungs- und Dosieranlagen bei der Verarbeitung möglich.

**Patentansprüche**

1. Verwendung einer Verbindung der Formel (1)

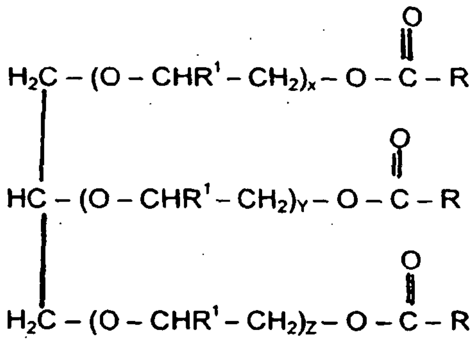

(1),

in der
die Reste $R^1$ gleich oder verschieden und unabhängig voneinander ausgewählt sind unter H und $CH_3$;
die Reste R gleich oder verschieden und unabhängig voneinander ausgewählt sind unter C7-C19-Fettsäureresten, und

$$x + y + z = 5 - 50,$$

zur permanenten Hydrophilierung von Polyolefin und/oder Polyester enthaltenden Materialien.

2. Verwendung nach Anspruch 1, wobei das Polyolefin ein Homo- oder Copolymer auf Ethylen- oder Propylen-Basis ist.

3. Verwendung nach einem der vorhergehenden Ansprüche, wobei das Material eine Faser oder ein Flächengebilde ist.

**Claims**

1. Use of a compound of formula (1)

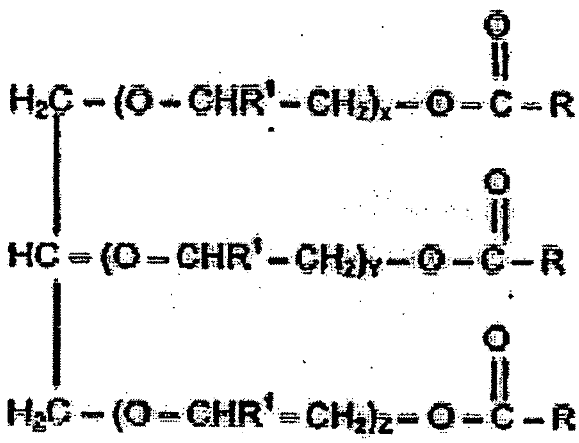

(1)

wherein
residues R' are selected from H and $CH_3$ to be the same as or different and independent from one another,

residues R are selected from C7-C19 fatty acid residues to be the same as or different and independent from one another, and

$$x + y + z = 5 - 50,$$

for the permanent hydrophilising of polyolefin- and/or polyester-containing materials.

2. Use according to claim 1 wherein the polyolefin is an ethylene- or propylene-based homopolymer or copolymer.

3. Use according to one of the preceding claims wherein the material is a fibre or a textile fabric.

**Revendications**

1. Utilisation d'un composé de formule (1) :

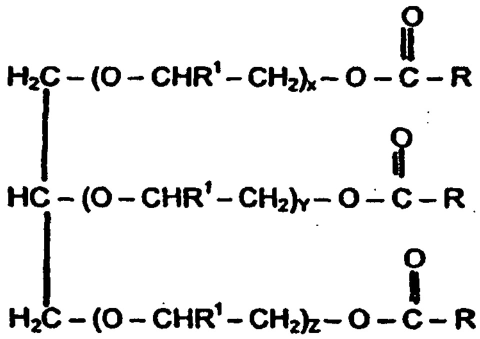

(1)

dans laquelle
les radicaux $R^1$, identiques ou différents et indépendamment les uns des autres, sont choisis parmi H et $CH_3$ ;
les radicaux R, identiques ou différents et indépendamment les uns des autres, sont choisis parmi les radicaux d'acides gras en $C_7$ et $C_{19}$, et

$$x + y + z = 5 - 50,$$

pour l'hydrophilisation permanente des matériaux contenant de la polyoléfine et/ou du polyester.

2. Utilisation selon la revendication 1, dans laquelle la polyoléfine est un homo- ou copolymère à base d'éthylène ou de propylène.

3. Utilisation selon l'une quelconque des revendications précédentes, dans laquelle le matériau est une fibre ou un non tissé.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10015554 A1